# EUROPÄISCHE PATENTANMELDUNG

(11) **EP 4 425 507 A1**
(43) Veröffentlichungstag der Anmeldung: **04.09.2024**
(21) Anmeldenummer: 23159723.8
(22) Anmeldetag: 02.03.2023
(51) Int. Cl.: G16H 70/60

(54) **SYSTEM ZUR IDENTIFIKATION VON MEDIZINISCHEN DATEN, COMPUTERIMPLEMENTIERTES VERFAHREN DAFÜR, COMPUTERPROGRAMMPRODUKT SOWIE EIN COMPUTERLESBARES SPEICHERMEDIUM**

(71) Anmelder: ACMIT Gmbh, 2700 Wiener Neustadt (AT)
(72) Erfinder: Dellantoni, Nikolaus, 2504 Sooss (AT); Bernhard, Nussbaumer, 2721 Bad Fischau (AT)
(74) Vertreter: IPrime Künsch Patentanwälte GmbH

(57) **Zusammenfassung**

Die Erfindung betrifft ein System 20 zur Identifikation von medizinischen Daten mD auf Basis von mindestens einer Zieldefinition 22 und umfasst ein Datenspeichermedium 25 mit mehreren medizinischen Daten mD zu menschlichen Körpern, ein Datenauswertemodul 30 mit einer Recheneinheit 32, eine Eingabeeinrichtung 40 zum Eingeben mindestens einer Zieldefinition 22, wobei eine Eingabe der mindestens einen Zieldefinition 22 das Datenauswertemodul 30 dazu instruiert, mindestens die folgenden Schritte auszuführen: Analysieren der mindestens einen Zieldefinition 22 in der Recheneinheit 32, Auswählen von medizinischen Daten mD aus dem Datenspeichermedium 25 mit einem Auswahlalgorithmus AA, Überprüfen einer vorgegebenen Vollständigkeit der medizinischen Daten mD mit einer gewünschten medizinischen Relevanz im Datenauswertemodul 30 basierend auf der analysierten Zieldefinition 23, und Bereitstellen der ausgewählten und/oder überprüften Daten D an einer Schnittstelle 45 für einen Benutzer. Weiters betrifft die Erfindung ein computerimplementiertes Verfahren sowie ein computerlesbares Medium.

## Beschreibung

Die vorliegende Erfindung betrifft ein System zur Identifikation von medizinischen Daten gemäss dem Patentanspruch 1, ein computerimplementiertes Verfahren gemäss dem Patentanspruch 13, ein Computerprogrammprodukt gemäss dem Patentanspruch 14 sowie ein computerlesbares Speichermedium gemäss dem Patentanspruch 15.

### Technologischer Hintergrund

Heutzutage werden tendenziell mehr medizinische Untersuchungen an Patienten als erforderlich durchgeführt, einfach deswegen, weil man vorher schwer abschätzen kann, ob man vielleicht etwas Ungewöhnliches findet, das die Diagnose beeinflusst. Immer wieder werden auch ungeeignete Untersuchungen durchgeführt, dies teilweise wegen Überlastung oder mangelnder Erfahrung des zuständigen Arztes. In der Vergangenheit wurde die Krankenakte jedes Patienten als physischer Papierordner lokal in einem Krankenhaus geführt. Die Notizen in den Patientenakten umfassten zum Beispiel einen Bericht über alle Vitalfunktionen, oder Testergebnisse und/oder andere klinische Daten, die während des Arztbesuches gesammelt wurden, oder eine oder mehrere vom Arzt festgestellte Diagnosen. Häufig diktierte der Arzt die Notiz mündlich in ein Audioaufzeichnungsgerät. Später wurden die Notizen der Patientenakte elektronisch auf ein Speichermedium abgelegt.

Bei Verdacht auf unnatürliche Todesursache werden bei verstorbenen Personen oftmals Autopsien bzw. Leichenschauen durchgeführt, um den Leichnam untersuchen und die Todesursache feststellen zu können. Dabei werden unter Zuhilfenahme von diagnostischen Untersuchungsmethoden medizinische Daten aus dem menschlichen Körper durch beispielsweise die Durchführung von Biopsien beim Leichnam oder die Anfertigung von Bildaufnahmen vom Leichnam generiert. Die dabei generierten medizinischen Daten aus dem menschlichen Körper wurden bisher rein für die Bestimmung der Todesursache einer Person und lokal im Krankenhaus genutzt. Dabei gäbe es eine Vielzahl an Nutzungsmöglichkeiten der medizinischen Daten, wenn man diese entsprechend aufbereiten und mit der Patientenakte verknüpfen könnte. Damit könnte man Stakeholdern, wie beispielsweise Medizintechnikunternehmen, gerichtlich beauftragte Sachverständige, die Pharmaindustrie und andere, welche derartige medizinische Daten benötigen, beliefern. Dabei ist die Qualität der erhobenen Daten im Hinblick auf die Zufriedenheit und Anforderungen der verschiedenen Stakeholder von hoher Relevanz.

Aus dem Stand der Technik ist die US 2014337057 A1 bekannt. Diese offenbart eine Vorrichtung zur Abschätzung der Todesursache, umfassend einen Abschnitt zur Erfassung diagnostischer Informationen, einen Abschnitt zur Erfassung der Krankheits-/Wundgeschichte und einen Abschnitt zur Abschätzung der Todesursache. Der Abschnitt zum Erfassen von Diagnoseinformationen ist so konfiguriert, dass er Diagnoseinformationen über Bilddaten eines Körpers erfasst. Der Abschnitt zum Erfassen der Krankheits-/Wundgeschichte ist so konfiguriert, dass er eine Diagnose/Behandlungsgeschichte des Körpers vor dem Tod des Körpers erfasst. Der Abschnitt zur Abschätzung der Todesursache ist so konfiguriert, dass er eine direkte Todesursache des Körpers und eine ursprüngliche Todesursache auf der Grundlage der erfassten Diagnoseinformationen und der erfassten Diagnose-/Behandlungshistorie abschätzt.

Nachteilig an dieser bekannten Lösung ist, dass die Bereitstellung der Daten nur auf einen menschlichen Körper abgestimmt ist.

Aus dem Stand der Technik ist die KR 101875306 B1 bekannt. Diese offenbart ein System zur Bereitstellung von Krankheitsinformationen, das medizinische Begriffscluster verwendet. Das System zur Bereitstellung von Krankheitsinformationen baut klinische und kausale Beziehungen zwischen medizinischen Begriffen auf, die in einem medizinischen Datenserver und einem Informationsbereitstellungsserver gespeichert sind, und baut Dokumentencluster mit Bezug auf Krankheiten durch die klinischen und kausalen Beziehungen auf, um die Suche nach Krankheiten durch Abfragen zu ermöglichen. Das System baut zur Bereitstellung von Krankheitsinformationen die klinischen und kausalen Beziehungen auf, die sich auf Informationen wie Symptome, Untersuchungen und Behandlungen von Krankheiten beziehen, um medizinische Informationen in Übereinstimmung mit Krankheiten leicht zu erkennen. Außerdem gruppiert das System zur Bereitstellung von Krankheitsinformationen die medizinischen Daten durch Bildung von Krankheitsclustern und ähnlichen Clustern, um verschiedene und genaue medizinische Informationen entsprechend den Abfrageinformationen durch Gruppierung ähnlicher Krankheiten bereitzustellen.

Aus dem Stand der Technik ist auch die EP 2673722 A1 bekannt. Diese offenbart ein computerimplementiertes Verfahren zum Erzeugen diskreter strukturierter Datenelemente für einen elektronischen Datensatz, aufweisend: Empfangen eines Originaltextes, der eine Darstellung einer von einem Kliniker vorgesehenen Schilderung eines Patientenkontaktes ist; Umformatieren des Originaltextes, um einen formatierten Text zu erzeugen, Extrahieren von einen oder mehreren klinischen Fakten aus dem formatierten Text, wobei der Schritt des Extrahierens ein Verwenden einer Kliniksprachenverständnismaschine aufweist, um den formatierten Text zu parsen, um klinische Begriffe in einem Lexikon einer Kliniksprache zu identifizieren, Konzepte zu identifizieren, die mit den klinischen Begriffen in einer formalen Ontologie verknüpft sind, und basierend auf der Sprachkenntnis in der formalen Ontologie Fakten zu extrahieren; Halten einer Verknüpfung zwischen jedem Fakt und dem entsprechenden Teil des Originaltextes; Anzeigen der einen oder mehreren klinischen Fakten in einem Faktenfeld auf einer grafischen Benutzerschnittstelle; als Reaktion auf eine Auswahl eines der im Faktenfeld angezeigten Fakten von einem Benutzer, Anzeigen eines Hinweises auf den entsprechenden Teil des Originaltextes, aus dem der ausgewählte Fakt einem Benutzer auf einer grafischen Benutzerschnittstelle extrahiert wurde, wodurch der Benutzer extrahierte Fakten unter Bezug auf den Originaltext prüfen kann und ein oder mehrere Änderungen an einen oder mehreren der extrahierten Fakten machen kann, um einen Satz klinischer Fakten für den Patientenkontakt zu schaffen; Speichern des Satzes klinischer Fakten entsprechend dem Patientenkontakt mit extrahierten Fakten und irgendwelchen vom Benutzer geschaffenen geänderten Fakten als diskrete strukturierte Datenelemente in einem elektronischen medizinischen Datensatz.

Nachteilig an diesen bekannten Lösungen ist, dass die Bereitstellung der Daten zu Krankheiten nur auf einen Patientenkontakt und nur lokal in einem Krankenhaus erfolgt.

### Darstellung der Erfindung

Eine Aufgabe der Erfindung besteht darin mindestens einige der Nachteile des Standes der Technik zu vermeiden. Insbesondere soll ein verbessertes, intelligentes Identifikationssystem geschaffen werden, welches bevorzugt die notwendigen Daten für eine medizinische Fragestellung aus einem grossen unabhängigen Datenpool zuverlässig bereitstellen kann. Es soll ein verbessertes computerimplementiertes Verfahren geschaffen werden, welches die notwendigen Daten zuverlässig auswählt und bereitstellt, sowie ein Computerprogrammprodukt und ein computerlesbares Medium dafür.

Diese Aufgabe wird durch die Merkmale der unabhängigen Patentansprüche gelöst. Vorteilhafte Weiterbildungen sind in den Figuren und in den abhängigen Patentansprüchen dargelegt.

Ein erfindungsgemässes System zur Identifikation von medizinischen Daten auf Basis von mindestens einer Zieldefinition umfasst ein Datenspeichermedium mit mehreren medizinischen Daten zu menschlichen Körpern, ein Datenauswertemodul mit einer Recheneinheit, welches zum Datenaustausch mit dem Datenspeichermedium verbunden ist, und eine Eingabeeinrichtung zum Eingeben mindestens einer Zieldefinition, welche mit dem Datenauswertemodul zum Datenaustausch verbunden ist, wobei eine Eingabe der mindestens einen Zieldefinition das Datenauswertemodul dazu instruiert, mindestens die folgenden Schritte auszuführen:
a) Analysieren der mindestens einen Zieldefinition in der Recheneinheit,
b) Auswählen von medizinischen Daten aus dem Datenspeichermedium mit einem Auswahlalgorithmus, wobei die ausgewählten Daten verknüpft mit der mindestens einen analysierten Zieldefinition ausgewählt werden,
c) Überprüfen einer vorgegebenen Vollständigkeit der medizinischen Daten mit einer gewünschten medizinischen Relevanz im Datenauswertemodul basierend auf der analysierten Zieldefinition,
d) Bereitstellen der ausgewählten und/oder überprüften Daten an einer Schnittstelle zur Bereitstellung für einen Benutzer und/oder einem KI -Modul.

Damit ist ein verbessertes, intelligentes Identifikationssystem geschaffen, welches lediglich die notwendigen medizinischen Daten aus zumindest einer eingegebenen Zieldefinition zuverlässig bereitstellt und nur medizinische Daten mit einer gewünschten Relevanz bereitstellt. Die medizinischen Daten stammen bevorzugt von vielen unterschiedlichen Patienten aus unterschiedlichen Krankenhäusern, Ländern oder Kontinenten. Die mindestens eine Zieldefinition legt die medizinische Anfrage, welche datenbasiert beantwortet werden soll, fest. Die mindestens eine Zieldefinition hat somit einen massgeblichen Einfluss auf die Auswahl der zu berücksichtigenden Inhalte der einzelnen medizinischen Daten. Die mindestens eine Zieldefinition kann entweder durch einen Benutzer in der Eingabeeinrichtung eingegeben werden oder mithilfe eines physischen Speichermediums oder einem Datennetzwerk an die Eingabeeinrichtung übergeben werden. Das hier offenbarte System identifiziert jene medizinischen Daten aus dem Datenspeichermedium, welche hinsichtlich der eingegebenen Zieldefinition benötigt werden, um beispielsweise eine medizinische Anfrage eindeutig und unmissverständlich zu beantworten. Damit ist der Aufwand der Datenanalyse optimiert und lediglich relevante Daten werden bereitgestellt. Die Schnittstelle kann die Eingabeeinrichtung beinhalten. Neben menschlichen Körpern ist das hier offenbarte Verfahren auch auf andere Lebewesen, wie beispielsweise tierische Körper, anwendbar.

Das Bereitstellen der ausgewählten und/oder überprüften Daten ermöglicht es dem Benutzer und/oder dem Kl (künstliche Intelligenz) Modul zu entscheiden, ob die Daten der eingegebenen Zieldefinition entsprechen, wobei die folgenden Schlussfolgerungen bzw. Erkenntnisse folgen: Entweder wurden die zur Erfüllung der mindestens einen Zieldefinition geeigneten medizinischen Daten mit der gewünschten Relevanz ausgegeben, oder es werden Informationen und/oder Instruktionen zum durchgeführten Vervollständigungsprozess bereitgestellt und/oder es werden Informationen zu zukünftigen Datenerhebungen mit einer Untersuchungseinrichtung bereitgestellt. Dies kann durch einen Benutzer veranlasst werden, indem beispielsweise die mindestens eine Zieldefinition angepasst wird, und/oder das KI-Modul passt die mindestens eine Zieldefinition selbstständig an und/oder liefert die Informationen und/oder Instruktionen zum Vervollständigungsprozess. Beispielsweise gibt die analysierte Zieldefinition vor, dass das Bereitstellen der medizinischen Daten im Schritt d) nur erfolgen kann, wenn die Anzahl der medizinischen Daten mit der gewünschten medizinischen Relevanz einen vorgegebenen Relevanz-Schwellwert, bevorzugt 98%, erreicht ist. Sollte der vorgegebene Schwellwert nicht erreicht werden, dann werden Instruktionen zum Vervollständigungsprozess an der Schnittstelle bereitgestellt und/oder es werden Informationen bzw. Instruktionen zu zukünftigen Datenerhebungen bereitgestellt. Damit ist ein sich selbst verbesserndes intelligentes Identifikationssystem für medizinische Daten mit hoher Qualität geschaffen.

Die ausgewählten und/oder überprüften medizinischen Daten können wiederum als Trainingsdaten für das KI-Modul dienen, sodass das System intelligent und selbstlernend ist. Damit wird ein System zur Identifikation von medizinischen Daten geschaffen, welches eine hohe Datenqualität aufweist und die Auswahl der Daten mit hoher Qualität favorisiert, sodass die mindestens eine Zieldefinition schnell und optimiert bearbeitet wird. Insbesondere weist das Kl-Modul den medizinischen Daten eine medizinische Relevanz auf Basis der mindestens einen Zieldefinition zu und verknüpft somit die medizinischen Daten mit unterschiedlichen Zieldefinitionen. Das KI-Modul kann wiederum ausgebildet sein, den medizinischen Daten eine medizinische Relevanz zuzuordnen, sodass diese bei eine nachfolgenden Zieldefinition verbessert auswählbar sind.

Das KI-Modul kann eine Methode des Deep Leaming (mittels künstlicher neuronaler Netze) einsetzen, bei der mehrere Schichten künstlicher Neuronen die Eingangsgrößen (Merkmalsvektor) mit der Ausgangsgröße (Klassifikation, Regression...) verknüpfen. Ebenso können zahlreiche andere Machine Learning Methoden, Random Forest Algorithmen (randomisierte Entscheidungsbäume) oder Support Vector Machines (Schätzung mittels Stützvektoren im Vektorraum der Merkmalsvektoren) eingesetzt werden, insbesondere um den Rechenaufwand zu begrenzen. Das KI-Modul wird prinzipiell mit historischen medizinischen Daten und/oder mit Daten aus einer Expertendatenbank trainiert.

Die medizinischen Daten in dem Datenspeichermedium können beispielsweise gelabelte Daten oder Datensätze sein, und umfassen eine Identifikationsnummer, mit der sie einfach vom Auswahlalgorithmus erfassbar sind. Damit ist beispielsweise einfacher erkennbar, ob die medizinischen Daten von einem Mann, mit einem zugeordneten Geburtsjahr, Blutgruppe, Grösse, Geburtsort, und weiteren Daten stammen. Die gelabelten Daten sind typischerweise pseudonymisiert. Die Identifikationsnummer kann dazu verwendet werden, einen gesamten Datensatz bzw. eine Datenstruktur eines menschlichen Körpers auszuwählen, sodass die medizinischen Daten mit der gewünschten Relevanz einfach und schnell auffindbar sind.

Der Auswahlalgorithmus kann auf eine Datenbank, wie beispielsweise eine SQL-Datenbank, angewendet werden, um die medizinischen Daten einfach zu erhalten. Dabei kann die Information aus der analysierten Zieldefinition sequenziell im Auswahlalgorithmus angewendet werden, um die Abfrage zu beschleunigen. Vorteilhaft sind die medizinischen Daten bereits sortiert in dem Datenspeichermedium abgelegt, sodass das sequenzielle Abfragen bzw. Auswählen der medizinischen Daten weiter beschleunigt ist. Die Auswahl der medizinischen Daten wird dadurch vereinfacht und verringert die Kosten.

Der Auswahlalgorithmus kann als Gruppierungsalgorithmus arbeiten und n medizinische Daten oder Identifikationsnummern zu k Gruppierungen partitionieren, in denen jedes Datenobjekt oder jede Identifikationsnummer zu der Gruppe mit dem nächstgelegenen gelabelten Daten oder Datensatz gehört. Werte von n-1 < k < n+1 können jedoch je nach Art der mindestens einen analysierten Zieldefinition und der zugrunde liegenden medizinischen Daten variieren. Das Auswählen von medizinischen Daten aus dem Datenspeichermedium erfolgt mit einem Auswahlalgorithmus, wobei die ausgewählten Daten verknüpft mit der mindestens einen analysierten Zieldefinition ausgewählt werden.

In der Informatik ist Quickselect ein Auswahlalgorithmus, um das k- te kleinste Element in einer ungeordneten Gruppe zu finden. Dabei kann die Identifikationsnummer als k-tes Element verwendet werden. Quickselect hängt mit dem Sortieralgorithmus Quicksort zusammen. Wie Quicksort ist es in der Praxis effizient und hat eine gute durchschnittliche Leistung. Quickselect und seine Varianten sind die Auswahlalgorithmen, die in effizienten Real-World-Implementierungen gut einsetzbar sind.

Quickselect verwendet den gleichen Gesamtansatz wie Quicksort, wählt ein Element als Pivot aus und teilt die Daten basierend auf dem Pivot in zwei Teile auf, entsprechend kleiner oder größer als der Pivot. Anstatt jedoch in beide Seiten zu rekursieren, wie bei Quicksort, rekursiert Quickselect nur in eine Seite - die Seite mit dem gesuchten Element. Dies reduziert die durchschnittliche Komplexität beim Auswählen.

Ein anderes Beispiel für einen Auswahlalgorithmus ist ein Echo Algorithmus. Um den Echo-Algorithmus als Auswahlalgorithmus benutzen zu können, muss jeder Knoten im Algorithmus eine eigene Identifikation haben. Jeder Knoten startet irgendwann einen Echo-Algorithmus, wobei sowohl die Echos als auch die Explorer die Identifikation ihres Initiators mitführen. Knoten ignorieren alle Nachrichten, deren Initiator eine kleinere Identifikation hat als sie selbst. Wenn ein Initiator von allen seinen Nachbarn ein Echo mit seiner eigenen Identifikation erhält, weiß er, dass er gewonnen hat. Alle anderen Knoten wissen, dass sie verloren haben, wenn sie einen Explorer mit einer höheren Identifikation als sie selbst empfangen haben.

Insbesondere erfolgt das Analysieren der mindestens einen Zieldefinition in der Recheneinheit im Schritt a) auf Basis mindestens einer bereits hinterlegten, historischen Zieldefinition aus einer Expertendatenbank. Damit kann die Expertendatenbank bereits hinterlegte Zieldefinitionen bereitstellen, um die Auswahl der medizinischen Daten mit einer hohen Effizienz und Qualität durchzuführen. Dafür ist das Datenauswertemodul mit der Expertendatenbank zum Austausch von Daten verbunden.

Insbesondere ist der Auswahlalgorithmus dynamisch anpassbar, sodass die Auswahl der medizinischen Daten abhängig von der Information der analysierten Zieldefinition ist. Je nach Kenntnis der zumindest einen Zieldefinition sowie der bereits hinterlegten historischen Zieldefinitionen ist der Auswahlalgorithmus anpassbar, um die Rechenleistung und damit die Kosten im System zu optimieren.

Die Expertendatenbank kann eine selbstlernende Expertendatenbank sein, in welcher Zusammenhänge bzw. Verknüpfungen zwischen der in den Zieldefinitionen, bzw. in Segmenten der Zieldefinitionen oder historischen Zieldefinitionen formulierten Aussageforderung und der dazu ausgewerteten medizinischen Daten aus menschlichen Körpern hinterlegt sind. Dabei kann die Expertendatenbank neue Zusammenhänge bzw. Verknüpfungen generieren und Zeiger (Pointer) zu Datensätzen mit medizinischen Daten erzeugen, um schneller die gewünschten medizinischen Daten im Datenspeichermedium zu erhalten.

Insbesondere erfolgt beim Auswählen im Schritt b) ein Vergleich der ausgewählten Daten mit weiteren medizinischen Daten aus der Expertendatenbank. Damit ist die Effizienz in der Auswahl der medizinischen Daten weiter verbesserbar.

Insbesondere veranlasst im Schritt d) das Datenauswertemodul die Schnittstelle, die ausgewählten und/oder überprüften Daten an einer Benutzerschnittstelle auszugeben. Damit ist das erwartete Ergebnis zur mindestens einen Zieldefinition einem Benutzer direkt zugänglich und der Benutzer kann weitere Handlungen vornehmen oder Korrekturen in der Zieldefinition vornehmen, um zu den gewünschten vollständigen medizinischen Daten mit der gewünschten Relevanz zu kommen. Insbesondere umfasst die Benutzerschnittstelle eine Ausgabeeinheit mit einem Display, bevorzugt einen Touchscreen, sodass die ausgewählten und/oder überprüften Daten dem Benutzer bedienerfreundlich zur Verfügung stehen.

Bevorzugt werden der Eingabeeinrichtung Rahmenbedingungen als Daten oder Datensätze bei der Eingabe zur Verfügung gestellt, und insbesondere dem Auswahlalgorithmus zur Verfügung gestellt. Unter Rahmenbedingungen werden in dem vorliegenden Dokument Einschränkungen der allgemeinen Zieldefinition, z.B. auf bestimmte Personengruppen (Alter, Geschlecht, Anzahl, etc.) verstanden. Die Rahmenbedingungen können über die Allgemeinheit einschränkende Konkretisierungen, z.B. bestimmte Zielgruppen (Geschlecht, Alter, Gewicht, Probenmenge bzw. Anzahl an verstorbenen menschlichen Körpern), definieren. Damit wird das Auswählen mithilfe des Auswahlalgorithmus mengenmässig eingeschränkt sowie vereinfacht und die medizinischen Daten können rascher bzw. kostengünstiger bereitgestellt werden.

Vorzugsweise umfasst das Datenspeichermedium körperbezogene Daten, welche mit den medizinischen Daten zumindest eines menschlichen Körpers assoziiert sind. Diese körperbezogenen Daten sind einem bestimmten menschlichen Körper zugehörige Daten (mit/ohne Personenbezug) und umfassen beispielsweise Geschlecht, Alter, Grösse, Gewicht, Hauttyp, Augenfarbe, BMI, DNA - Informationen, Herkunftsland usw.

Insbesondere stammen die medizinischen Daten von einem verstorbenen menschlichen Körper, welche bevorzugt aus einem Autopsie-Prozess geschaffen werden. Unter medizinischen Daten bzw. Datensätzen von verstorbenen Körpern werden zum einen Autopsiedaten verstanden und zum anderen anonymisierte oder pseudonymisierte historische Körperdaten bzw. Daten aus einer Patientenakte eines Verstorbenen, oder auch historische Datensätze, die die Krankengeschichte eines Verstorbenen und alle damit in Verbindung stehenden medizinischen Daten widerspiegeln. Darüber hinaus besteht mit dem hier offenbarten System die Möglichkeit, die angefertigten medizinischen Daten aus dem menschlichen Körper, die bisher rein für die Bestimmung der Todesursache genutzt wurden, auch anderweitig zu nutzen.

Bevorzugterweise umfasst die mindestens eine Zieldefinition mindestens eine Fragestellung. Dabei kann die Fragestellung aus Textbausteinen bestehen und/oder Abkürzungen und/oder Kennzahlen umfassen. Die Fragestellung kann medizinische Fachbegriffe und Fachabkürzungen umfassen und wird mithilfe der Recheneinheit analysiert. Dabei kann die Recheneinheit ein Texterkennungsprogramm umfassen, welche formatierte und unformatierte Textbausteine erkennt. Die Recheneinheit kann mit einem Register mit formatierten Textbausteinen verbunden sein, um die zuvor genannte Information aus der Fragestellung beispielsweise medizinischen Daten zuordnen zu können und diese zu analysieren. Die analysierte Zieldefinition wird anschliessend dem Auswahlalgorithmus zur Verfügung gestellt, um die medizinischen Daten aus dem Datenspeichermedium auszuwählen. Beispielsweise kann die mindestens eine Zieldefinition mehrere Fragestellungen umfassen, welche wie zuvor beschrieben, analysiert werden und insbesondere auch in Abhängigkeit voneinander analysiert werden. Insbesondere umfasst die mindestens eine Zieldefinition mindestens eine Fragestellung, welche mittels Analyse von medizinischen Daten bzw. Verknüpfungen mit den medizinischen Daten aus verstorbenen menschlichen Körpern beantwortbar ist.

Alternativ oder ergänzend umfasst die mindestens eine Zieldefinition medizinische Daten. Die medizinischen Daten können vom Benutzer in einer Vorlage für eine Zieldefinition in der Eingabeeinrichtung eingegeben werden, oder von einem Speichermedium oder über ein Datennetzwerk eingelesen werden. Beispielhaft können erhobene Leberwerte auf eine Langzeitmedikamenteneinnahme hindeuten, wobei die Erhebung von weiteren medizinischen Daten in der mindestens einen Zieldefinition empfohlen wird, da ein Pharmaunternehmen diese medizinischen Daten für eine Studie benötigt. Die entsprechenden erhobenen Leberwerte können als medizinische Daten in die mindestens eine Zieldefinition eingegeben werden. Alternativ oder ergänzend umfasst die mindestens eine Zieldefinition körperbezogene Daten, welche ergänzend für Studien verwendbar wären und beispielsweise vom Benutzer der mindestens einen Zieldefinition hinzugefügt werden.

Alternativ oder ergänzend umfasst die mindestens eine Zieldefinition mindestens ein medizinisches Datengewinnungsverfahren. Es wäre denkbar, dass bei einem CT-Scan eines Leichnams ein künstliches Hüftgelenk detektiert wird, wobei die Erhebung von erweiterten medizinischen Daten mit einem weiteren medizinischen Datengewinnungsverfahren in der mindestens einen Zieldefinition empfohlen wird, da ein Medizintechnikunternehmen diese medizinischen Daten für interne Zwecke bzw. die Weiterentwicklung ihrer Hüftgelenkprothesen benötigt. Damit können Daten zur Verfügung gestellt werden, welche beispielsweise ein Zulassungsverfahren einer neuartigen Hüftgelenksprothese vereinfachen können, sodass Medizintechnikunternehmen mit ihren Produkten einfacher und kostengünstiger durch Zulassungsverfahren kommen.

Als weiteres Beispiel kann nach der automatisierten Auswertung von erstellten Bildaufnahmen der Haut eines menschlichen Körpers die Erkennung von Hämatomen auf eine Fremdeinwirkung hindeuten, wobei in weiterer Folge eine erweiterte forensische Datenerhebung am Leichnam empfohlen wird, um eine unnatürliche Todesursache ausschliessen zu können.

Nachfolgend folgt ein nicht abschliessender Überblick über mögliche medizinische Daten, die beim Verstorbenen erhoben werden können: Ganzkörper CT Bilddaten in feiner Auflösung (aufgrund Risikobetrachtung, insbesondere einer hohen Strahlenbelastung, kann dies bei Lebenden kaum durchgeführt werden), Gewebs- und Flüssigkeitsproben, MR-Bilddaten, Oberflächenscans, Angiografie, sowie alle Daten über den Verstorbenen z.B. aus einer über die Lebensjahre gefüllten Patientenakte bzw. einer elektronischen Gesundheitsakte.

Vorzugsweise sind die ausgewählten medizinischen Daten in einer Datenstruktur im Datenspeichermedium abgelegt. Damit sind die ausgewählten medizinischen Daten einfach auffindbar und verbessert mit der mindestens einen Zieldefinition verknüpfbar. Beispielsweise sind die medizinischen Daten in Cluster zusammengefasst, sodass diese einfach mit der Expertendatenbank verknüpfbar sind. Alternativ oder ergänzend sind die körperbezogenen Daten in einer Datenstruktur im Datenspeichermedium abgelegt. Damit sind auch diese Daten einfach auffindbar und verbessert mit der Expertendatenbank verknüpfbar.

Insbesondere umfasst das Datenspeichermedium ein hierarchisches Kategorienschema, mittels welchem die medizinischen Daten aus menschlichen Körpern strukturiert abgelegt werden können, wobei jeder Kategorie, bzw. Kategorieebene eine eindeutige Kategoriebezeichnung (z.B. Leukozyten), bzw. Kategorieebenenbezeichung (z.B. Granulozyten) und/oder ein Wert bzw. Werteintervall zugeordnet ist, sodass eine gut aufgeschlüsselte Datenstruktur entsteht, welche einfach mit dem Auswahlalgorithmus durchforstbar ist, um die medizinischen Daten mit der gewünschten Relevanz aufzufinden.

Bevorzugterweise ist die Recheneinheit ausgebildet, im Schritt a) ein Datenabfragemuster mithilfe der analysierten Zieldefinition zu erzeugen. Dies ermöglicht insbesondere, eine für eine gewünschte Relevanz abhängige Erhebung von medizinischen Daten aus einem verstorbenen menschlichen Körper zur Verfügung zu stellen, wobei das Datenabfragemuster auf Grundlage bestimmter Fragestellungen generiert wird und medizinische Daten aus verstorbenen menschlichen Körpern aus dem Datenpool des Datenspeichermediums ausgewählt und mit dem jeweiligen Datenabfragemuster verglichen werden. Wird dabei festgestellt, dass der Datenpool im Datenspeichermedium nur unzureichende medizinische Daten liefern kann, werden in einer weiteren Datenerhebung mit einer Untersuchungseinrichtung die zur Beantwortung der Fragestellung nötigen zusätzlichen medizinische Daten aus dem/den verstorbenen menschlichen Körper(n) erhoben.

Die Datenabfragemuster definieren sich bevorzugt aus Fragestellungen, medizinischen und/oder körperbezogene Daten und Erhebungsverfahren bzw. Untersuchungsverfahren, d.h. beispielsweise: (a) einer Liste von Fragestellungen, die von unterschiedlichen Stakeholdern vorgegeben werden bzw. die aus einer Datenanalyse folgend neu entstehen, und/oder b) einer Liste von zu erhebenden medizinischen Daten je Fragestellung, die dem aus medizinischer Sicht notwenigen Stand-der-Technik folgend laufend aktualisiert wird, und/oder (c) einer Liste von Verfahren zur Erhebung der medizinischen Daten, die im Hinblick auf den Kundennutzen für die Stakeholder laufend optimiert wird.

Vorteilhaft gibt es eine Liste von Fragestellungen mit zugehörigen zu erhebenden medizinischen und/oder körperbezogenen Daten, wobei einerseits die Liste der Fragestellungen laufend erweitert wird und andererseits die je Fragestellung zugehörigen zu erhebenden Daten laufend aktualisiert (verbessert) werden. Je zu erhebendem Datensatz kann es eine oder mehrere Verfahren geben, um diese Daten zu gewinnen. Auch diese(s) Verfahren wird (werden) laufend verbessert (optimiert).

Im Falle, dass die Übereinstimmung zwischen dem Datenabfragemuster und den medizinischen Daten aus menschlichen Körpern nicht vollständig ist bzw. nicht vorhanden ist, können die aktuellen medizinischen Daten aus menschlichen Körpern mit historischen Daten aus menschlichen Körpern angereichert werden. Die vorgegebene Vollständigkeit der medizinischen Daten lässt sich insbesondere verbessern, indem die unvollständigen Daten zuerst mit Daten der Patientenakte angereichert werden und, wenn notwendig in einem nächsten Schritt auch medizinische Daten aus anderen menschlichen Körpern, die aufgrund statistischer Kriterien eine ausreichend hohe Ähnlichkeit mit dem betroffenen Fall besitzen, weiter angereichert werden. Auch dieser Prozess ist laufend optimierbar und mithilfe des KI-Moduls weiter verbesserbar.

Medizinisch relevante Daten können somit auch solche sein, die einen ausreichenden Übereinstimmungsgrad mit dem Datenabfragemuster haben. Der Relevanzfaktor bzw. deren Schwellwert ergibt sich aus:
(a) der Qualität der erhobenen medizinischen Daten im Hinblick auf eine Zufriedenheit des Stakeholders mit der Aussagekraft der medizinischen Daten, d.h. der Stakeholder konnte seine Frage ausreichend beantworten. Andernfalls müsste die mindestens eine Zieldefinition der zu erhebenden medizinischen Daten zur Frage des Stakeholders adaptiert werden. Der Relevanzfaktor ist spezifisch für die Fragestellung des Stakeholders. Die Qualität kann mittels verschiedener Faktoren beschrieben werden. Beispielsweise wurden nicht die richtigen medizinischen Daten erhoben oder die identifizierten medizinischen Daten waren zu ungenau, etc., und/oder
(b) dem Aufwand zur Datenidentifikation, um Zeitaufwand und Kosten zu optimieren.

Vorzugsweise ist die Recheneinheit ausgebildet, die medizinischen Daten nach dem Schritt a) gemäss eines Kategorienschemas in einzelne Kategorien des Kategorienschemas zu zerlegen und somit ein Datenabfragemuster zu erzeugen. Das Kategorienschema kann dabei als zentrales Element der inhaltlichen Datenanalyse dienen. Über das Kategorienschema werden die medizinischen und/oder körperbezogene Daten festgelegt, deren Berücksichtigung zur Analyse der mindestens einen Zieldefinition die Basis bilden, wobei das Kategorienschema hierarchisch aufgebaut sein kann. Beispielsweise Kategorie Ebene 1: Blutbild; Kategorie Blutbild - Ebene 2: Leukozyten, Erythrozyten, Hämoglobin; und Kategorie Blutbild - Leukozyten - Ebene 3: Granulozyten, Monozyten, Lymphozyten.

Das Identifikationssystem ermöglicht es bevorzugt, aus einer Anfrage bzw. mindestens einer Zieldefinition ein Datenabfragemuster zu erzeugen, welches es ermöglicht, mittels einer selbstlernenden Expertendatenbank ähnliche Datenabfragemuster zu identifizieren, welche die mindestens eine Zieldefinition erfüllen. Dabei enthält die Expertendatenbank Verknüpfungen zwischen der in der mindestens einen Zieldefinition formulierten Aussageforderung und der dazu auszuwertenden Kategorien der im Identifikationssystem hinterlegen Datensätze.

Insbesondere ist die Recheneinheit ausgebildet zumindest die ausgewählten medizinischen Daten in mindestens zwei Kategorien einzuteilen. Damit lassen sich die ausgewählten medizinischen Daten unterschiedlich klassifizieren. Alternativ oder ergänzend ist die Recheneinheit ausgebildet die mindestens eine Zieldefinition in mindestens zwei Kategorien einzuteilen. Dabei kann die Recheneinheit die mindesten eine Zieldefinition und deren Rahmenbedingungen analysieren und gemäss des Kategorienschemas in einzelne Kategorien des Kategorienschemas zerlegen und somit ein Datenabfragemuster erzeugen.

Bevorzugterweise umfasst das Datenabfragemuster einen Identifikationsteil, welcher die relevanten Daten anhand der Kategorien identifiziert, sodass eine reproduzierbare Klassifizierung erfolgen kann.

Insbesondere umfasst das Datenabfragemuster einen Auswerteteil, welcher die medizinischen Daten der identifizierten Kategorien auswertet. Dabei werden die Werte der Daten in den identifizierten Kategorien mit der Recheneinheit ausgewertet. Alternativ oder ergänzend wertet der Auswerteteil Datenintervalle der identifizierten Kategorien aus.

Insbesondere geht die Recheneinheit dabei mehrstufig vor, wobei in einem ersten Schritt die durch die mindestens eine Zieldefinition und/oder medizinischen Daten definierten Kategorien identifiziert werden und in einem zweiten Schritt die zur Beantwortung der mindestens einen Zieldefinition relevanten Werteintervalle festgelegt werden. Dabei kann die Recheneinheit ausgebildet sein, die Identifikation der Kategorien selbstlernend vorzunehmen.

Vorzugsweise ist der Auswahlalgorithmus ausgebildet, das erzeugte Datenabfragemuster mit den im Datenspeichermedium hinterlegten medizinischen Daten zu vergleichen, um die vorgegebene Vollständigkeit der Daten im Schritt c) zu überprüfen. Dabei kann der Auswahlalgorithmus die Datenabfragemuster mit den im Datenspeichermedium hinterlegten, kategorisierten Daten bzw. Datensätze aus menschlichen Körpern vergleichen und (a) die medizinischen Daten bzw. Datensätze der menschlichen Körper mit einer hinreichenden, z.B. 100%-igen, Übereinstimmung ausgeben, oder (b) die medizinischen Daten bzw. Datensätze der menschlichen Körper mit einer nicht hinreichenden Übereinstimmung, bezüglich der Kategorisierung ausgeben , oder (c) bei einer unzureichenden Vollständigkeit eine zukünftige Identifikation bzw. Datenerhebung gemäss eines Datenabfragemuster auslösen, wobei sich die zukünftige Datenerhebung beispielsweise entweder auf einem nächsten Autopsieschritt einer laufenden Autopsie und/oder auf die Datenerhebungen von zukünftigen Autopsien bezieht. Dabei kann die Definition der zukünftigen Datenerhebung entweder mehrere alternative medizinische Daten enthalten, oder in Abhängigkeit der Auswahl mit dem Auswahlalgorithmus aus der Recheneinheit eine Reduzierung der erhobenen medizinischen Daten nach sich ziehen.

Bevorzugterweise ist das Überprüfen einer vorgegebenen Vollständigkeit der medizinischen Daten mit einer gewünschten medizinischen Relevanz im Schritt c) mit dem erzeugten Datenabfragemuster assoziiert. Damit kann bereits anhand des erzeugten Datenabfragemusters erkannt werden, ob die vorgegebene Vollständigkeit der medizinischen Daten überhaupt erreichbar ist.

Vorzugsweise ist das Datenauswertemodul ausgebildet, auf Basis der mindestens einen Zieldefinitionen in Kombination mit Information aus der Expertendatenbank die zur Beantwortung der analysierten Zieldefinition notwendigen Datensatzfelder auszuwerten. Eine schnelle Analyse der mindestens einen Zieldefinition ist mithilfe der Expertendatenbank möglich, sodass insgesamt eine reproduzierbare Auswahl der medizinischen Daten möglich ist.

Bevorzugterweise ist das Datenauswertemodul ausgebildet, eine Datenstruktur mit medizinischen Daten zu ergänzen. Damit verbessert sich das System selbst und zukünftige Zieldefinitionen sind schneller und mit einer erhöhten Qualität analysierbar.

Alternativ oder ergänzend ist das Datenauswertemodul ausgebildet, eine Datenstruktur mit körperbezogenen Daten zu ergänzen, sodass die Qualität weiter verbessert ist.

Insbesondere ist das Datenauswertemodul ausgebildet, diese vervollständigten medizinischen Daten in einer weiteren Datenstruktur im Datenspeichermedium abzuspeichern, und insbesondere anonymisiert abzuspeichern. Damit wird der Datenpool im Datenspeichermedium inhärent mit einer verbesserten Datenqualität angehäuft.

Vorteilhaft ist das Datenauswertemodul ausgebildet eine Gruppe der Datensätze von verstorbenen Körpern mit einer nicht hinreichenden Übereinstimmung mit einer Gruppe der Datensätze von verstorbenen Körpern mit einer hinreichenden Übereinstimmung unter Berücksichtigung der Datenabfragemuster zu vergleichen und bei erkannten inhärenten Datenabfragemustern die Datensätze der verstorbenen Körper mit einer nicht hinreichenden Übereinstimmung mittels eines mathematischen Modells, welches es erlaubt zu mindestens teilweise, die Werte oder Wertintervalle der unvollständigen Kategorien / Daten zu vervollständigen. Dabei kann das Datenauswertemodul auf das obenstehende KI-Modul zugreifen oder eine eigene künstliche Intelligenz umfassen, welche beispielsweise in der Recheneinheit angeordnet ist. Damit ist das Datenauswertemodul eine selbstlernende Einheit, welche die Daten automatisch vervollständigen kann. Insbesondere greift das Datenauswertemodul dabei auf die Expertendatenbank zu und tauscht die medizinischen und/oder körperbezogenen Daten aus.

Insbesondere ist ein Kundenzufriedenheitsinformationsmodul vorhanden, welches sich auf die Kundenzufriedenheit bezüglich der Auswahlqualität der medizinischen Daten bezieht und kundezufriedenheitsbasierte Veränderungen entweder in der Expertendatenbank oder in dem Datenauswertemodul vornimmt.

Vorzugsweise ist mindestens eine medizinische Untersuchungseinrichtung vorhanden, welche mit der Schnittstelle zum Austausch von Daten und/oder von Steuerbefehlen verbunden ist. Alternativ oder ergänzend ist die mindestens eine medizinische Untersuchungseinrichtung mit der Eingabeeinrichtung zum Austausch von Daten und/oder von Steuerbefehlen verbunden. Damit ist eine Rückkopplung vorhanden, welche im Falle einer unzureichenden Datenbasis dem Autopsiegerät, zieldefinitionsabhängig mitteilt, welche medizinischen Daten zusätzlich, bzw. nicht mehr innerhalb einer Standardautopsie erhoben werden müssen.

Alternativ oder ergänzend ist mindestens eine medizinische Untersuchungseinrichtung vorhanden, welche mit einem KI-Modul zum Austausch von Daten und/oder von Steuerbefehlen verbunden ist. Das KI-Modul kann somit die medizinische Untersuchungseinrichtung steuern und somit reproduzierbar medizinische Daten mit einer gewünschten medizinischen Relevanz erzeugen.

Bevorzugterweise ist das Datenauswertemodul ausgebildet, Steuerbefehle für zumindest eine medizinische Untersuchungseinrichtung zu erstellen. Damit kann das intelligente Identifikationssystem direkt auf eine medizinische Untersuchungseinrichtung zugreifen und diese steuern, sodass medizinische Daten erhebbar sind, welche eine gewünschte medizinische Relevanz aufweisen.

Ein erfindungsgemässes computerimplementiertes Verfahren zur Identifikation von medizinischen Daten auf Basis von Zieldefinitionen umfasst mindestens die folgenden Schritte:
a) Bereitstellen von einzelnen oder mehreren medizinischen Daten aus einem Datenspeichermedium,
b) Erhalten von mindestens einer Zieldefinition in einem Datenauswertemodul mit einer Recheneinheit,
c) Analysieren der mindestens einen Zieldefinition in der Recheneinheit,
d) Auswählen von medizinischen Daten aus dem Datenspeichermedium mit einem Auswahlalgorithmus, wobei die ausgewählten Daten verknüpft mit der mindestens einen Zieldefinition ausgewählt werden,
e) Überprüfen einer vorgegebenen Vollständigkeit der medizinischen Daten mit einer gewünschten medizinischen Relevanz im Datenauswertemodul basierend auf der analysierten Zieldefinition,
f) Bereitstellen der ausgewählten und/oder überprüften Daten an einer Schnittstelle zur Bereitstellung für einen Benutzer und/oder einem KI-Modul, wobei insbesondere das Datenauswertemodul die Schnittstelle veranlasst, die ausgewählten und/oder überprüften Daten an einer Benutzerschnittstelle auszugeben.

Damit ist ein verbessertes, intelligentes computerimplementiertes Identifikationsverfahren geschaffen, welches die notwendigen medizinischen Daten aus einer eingegebenen Zieldefinition zuverlässig bereitstellt und medizinische Daten mit einer gewünschten Relevanz bereitstellt. Bevorzugte Ausführungsformen im computerimplementierten Verfahren sind bereits im zuvor beschriebenen erfindungsgemässen System offenbart und sind als Verfahrensschritte ausführbar.

Ein erfindungsgemässes Computerprogrammprodukt umfassend Programmbefehle, welche ausgebildet sind, zumindest ein hier vorliegend beschriebenes Verfahren auszuführen. Das Computerprogrammprodukt kann an einer Recheneinheit ausgeführt werden, und somit die Programmbefehle schrittweise abarbeiten, um die ausgewählten und/oder überprüften Daten an einer Schnittstelle bereitzustellen. Diese können dann einem Benutzer, einem KI-Modul bereitgestellt werden, um insbesondere eine Untersuchungseinrichtung zu steuern.

Ein erfindungsgemässes computerlesbares Speichermedium umfasst das zumindest eine Computerprogrammprodukt, das bei der Ausführung durch zumindest eine Recheneinheit dieses veranlasst, zumindest ein vorliegend beschriebenes Verfahren durchzuführen.

Das hier vorliegend beschriebene System und Verfahren ermöglicht eine anforderungsbezogene bzw. kundenspezifisch optimale Erhebung von medizinischen Daten aus verstorbenen oder lebenden menschlichen Körpern durchzuführen. Dies gewährleistet bei der Erhebung von medizinischen Daten aus menschlichen Körpern eine möglichst gezielte und genaue Datenerhebung von relevanten Datensätzen, da beispielsweise auch körperbezogene Daten (Alter, Gewicht, Geschlecht etc.) vom System berücksichtigt werden.

Zudem ermöglicht ein solches System und Verfahren, die Datenerhebung gezielt zu steuern und zu kontrollieren. Dabei wird es möglich, insbesondere nur relevante Daten zu erheben und den Datenerhebungsprozess kontinuierlich anzupassen und zu optimieren.

In Zuge dessen können auch Kosten bei der Datenerhebung eingespart werden, da nicht alle, sondern nur relevante Daten erhoben werden. Dies erlaubt es, bei der Datenerhebung eine geringere Anzahl an CT-Scans, Bildaufnahmen oder Biopsie Proben zu entnehmen und anzufertigen.

Mit dem System und dem Verfahren ist eine Reduzierung des zeitlichen Aufwandes, der für die Datenerhebung nötig ist, möglich, da anstelle des üblichen Umfangs nur noch erforderliche bzw. relevante Daten bezüglich eines kundenspezifischen bzw. anwendungsspezifischen Falles bei der Datenerhebung aus menschlichen Körpern erhoben werden müssen.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Mittels der nachfolgenden Figuren wird anhand von Ausführungsbeispielen die Erfindung näher erläutert. Die Bezugszeichenliste ist Bestandteil der Offenbarung.

Positionsangaben, wie "oben", unten", "rechts" oder "links" sind jeweils auf die entsprechenden Darstellungen bezogen und sind nicht als einschränkend zu verstehen.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren, auch wenn sie dort im Zusammenhang mit anderen Merkmalen gezeigt sind und/oder vorstehend nicht genannt sind. Im Weiteren schliesst der Ausdruck "umfassen" und Ableitungen davon andere Elemente oder Schritte nicht aus. Ebenfalls schliesst der unbestimmte Artikel "ein" beziehungsweise "eine" und Ableitungen davon eine Vielzahl nicht aus. Die Funktionen mehrerer in den Ansprüchen aufgeführter Merkmale können durch eine Einheit erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen.

### Figurenbeschreibung

Die Figuren werden zusammenhängend und übergreifend beschrieben. Gleiche Bezugszeichen bedeuten gleiche Bauteile. Es zeigen
Fig. 1 : eine erste Ausführungsform des Systems zur Identifikation von medizinischen Daten auf Basis von einer Zieldefinition in einer schematischen Darstellung,
Fig. 2: eine zweite Ausführungsform des Systems zur Identifikation von medizinischen Daten auf Basis von einer Zieldefinition in einer schematischen Darstellung,
Fig. 3: eine dritte Ausführungsform des Systems zur Identifikation von medizinischen Daten auf Basis von einer Zieldefinition mit einer detaillierten schematischen Darstellung eines Datenauswertemoduls, und
Fig. 4 ein Flussdiagramm zu einem computerimplementierten Verfahren, ausführbar in einem System einer der Ausführungsformen gemäss den Figuren 1 bis 3.

### Ausführung der Erfindung

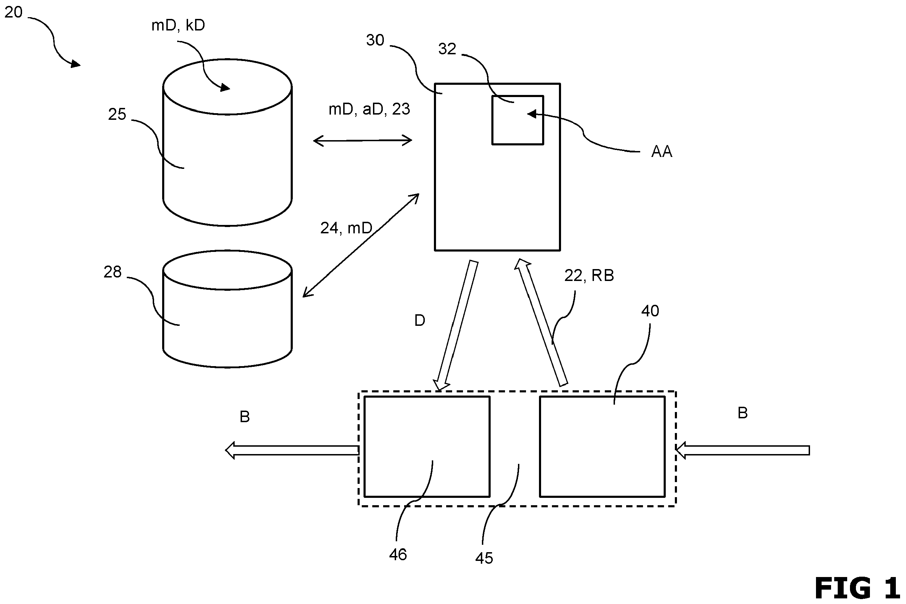

**Figur 1** zeigt eine Ausführungsform des Systems 20 zur Identifikation von medizinischen Daten auf Basis von einer Zieldefinition 22 umfassend ein Datenspeichermedium 25 mit einem Datenpool von mehreren medizinischen Daten mD zu menschlichen Körpern, ein Datenauswertemodul 30 mit einer Recheneinheit 32, welches zum Datenaustausch mit dem Datenspeichermedium 25 verbunden ist, eine Eingabeeinrichtung 40 zum Eingeben einer Zieldefinition 22, welche mit dem Datenauswertemodul 30 zum Datenaustausch verbunden ist, wobei eine Eingabe der Zieldefinition 22 das Datenauswertemodul 30 dazu instruiert, mindestens die folgenden Schritte auszuführen:
a) Analysieren der Zieldefinition 22 in der Recheneinheit 32,
b) Auswählen von medizinischen Daten mD aus dem Datenspeichermedium 25 mit einem Auswahlalgorithmus AA, wobei die ausgewählten Daten aD verknüpft mit der analysierten Zieldefinition 23 ausgewählt werden,
c) Überprüfen einer vorgegebenen Vollständigkeit der medizinischen Daten mD mit einer gewünschten medizinischen Relevanz im Datenauswertemodul 30 basierend auf der analysierten Zieldefinition 23,
d) Bereitstellen der ausgewählten und/oder überprüften Daten D an einer Benutzerschnittstelle 45 zur Bereitstellung für einen Benutzer B, wobei das Datenauswertemodul 30 eine Ausgabeeinheit 46 der Benutzerschnittstelle veranlasst, die ausgewählten und/oder überprüften Daten D auszugeben.

Die medizinischen Daten mD stammen von vielen unterschiedlichen lebenden und verstorbenen Patienten aus unterschiedlichen Krankenhäusern, Ländern oder Kontinenten und sind anonymisiert. Die Zieldefinition 22 wird durch einen Benutzer B in der Eingabeeinrichtung 40 eingegeben. Gleichzeitig werden vom Benutzer B Rahmenbedingungen als Daten zur Verfügung gestellt. Unter Rahmenbedingungen RB werden in dem vorliegenden Dokument Einschränkungen der allgemeinen Zieldefinition 22, z.B. auf bestimmte Personengruppen (Alter, Geschlecht, Anzahl, etc.) verstanden.

Das hier offenbarte System 20 identifiziert jene medizinischen Daten mD aus dem Datenspeichermedium 25, welche ausschliesslich hinsichtlich der Beantwortung oder Erfüllung der eingegebenen Zieldefinition 22 benötigt werden, um beispielsweise eine medizinische Fragestellung eindeutig und unmissverständlich zu beantworten.

Die medizinischen Daten mD in dem Datenspeichermedium 25 sind gelabelte Daten oder Datensätze, und umfassen eine Identifikationsnummer ID, mit der sie einfach vom Auswahlalgorithmus AA erfassbar sind. Der Auswahlalgorithmus AA kann auch medizinische Daten beispielsweise anhand der gelabelten Datensätze auswählen. Damit ist beispielsweise einfacher erkennbar, ob die medizinischen Daten mD von einem Mann, mit einem zugeordneten Geburtsjahr, Blutgruppe, Grösse, Geburtsort, und weiteren Daten stammen. Zusätzlich umfasst das Datenspeichermedium 25 körperbezogene Daten kD, welche mit den medizinischen Daten mD zumindest eines menschlichen Körpers assoziiert sind. Die körperbezogenen Daten kD umfassen Daten, wie beispielsweise Geschlecht, Alter, Grösse, Gewicht, Hauttyp, Augenfarbe, BMI, DNA - Informationen, Herkunftsland usw.

Das Datenauswertemodul 30 ist ausgebildet, eine Datenstruktur mit medizinischen Daten mD und mit körperbezogenen Daten kD vor dem Schritt d) zu ergänzen, sowie diese vervollständigten medizinische Daten in einer weiteren Datenstruktur im Datenspeichermedium 25 abzuspeichern.

In der vorliegenden Ausführungsform umfasst die Zieldefinition eine Fragestellung aus Textbausteinen und Kennzahlen. Die Textbausteine umfassen medizinische Fachbegriffe und Fachabkürzungen und werden mithilfe der Recheneinheit 32 analysiert. Die analysierte Zieldefinition 23 wird anschliessend dem Auswahlalgorithmus AA zur Verfügung gestellt, um die medizinischen Daten mD auszuwählen. Alternativ dazu kann die Zieldefinition auch medizinische Daten mD umfassen.

Das Analysieren der Zieldefinition 22 in der Recheneinheit 32 im Schritt a) erfolgt in diesem Beispiel auf Basis mindestens einer bereits hinterlegten, historischen Zieldefinition 24 aus einer Expertendatenbank 28, welche mit dem Datenauswertemodul 30 zum Austausch von Daten verbunden ist. Dabei erkennt der Auswahlalgorithmus AA in der Recheneinheit 32 die Zieldefinition 22 als eine bereits hinterlegte, historische Zieldefinition 24 und wählt die medizinischen Daten mD aus dem Datenspeichermedium 25 entsprechend aus. Anschliessend wird die Vollständigkeit der medizinischen Daten mD entsprechend dem Schritt c) überprüft.

Die Expertendatenbank 28 ist eine selbstlernende Expertendatenbank, in welcher Zusammenhänge bzw. Verknüpfungen zwischen der in den Zieldefinitionen 22, bzw. in Segmenten der Zieldefinitionen oder historischen Zieldefinitionen 24 formulierten Aussageforderung und der dazu ausgewerteten medizinischen Daten mD aus menschlichen Körpern hinterlegt sind. Dabei kann die Expertendatenbank 28 neue Zusammenhänge bzw. Verknüpfungen generieren und Zeiger (Pointer) zu Datensätzen mit medizinischen Daten mD erzeugen, um schneller zu den gewünschten medizinischen Daten mD im Datenspeichermedium 25 zu gelangen. Dabei ist das Datenauswertemodul 30 ausgebildet, auf Basis der Zieldefinitionen 22 in Kombination mit Information aus der Expertendatenbank 28 die zur Beantwortung der analysierten Zieldefinition 23 notwendigen Datensatzfelder auszuwerten.

Die Recheneinheit 32 ist ausgebildet, im Schritt a) ein Datenabfragemuster mithilfe der analysierten Zieldefinition 23 zu erzeugen, wobei Datenabfragemuster auf Grundlage bestimmter Fragestellungen generiert werden, und Daten aus dem Datenpool des Datenspeichermediums 25 ausgewählt und mit dem jeweiligen Datenabfragemuster verglichen werden. Somit ist das Überprüfen der vorgegebenen Vollständigkeit der medizinischen Daten mD mit der gewünschten medizinischen Relevanz im Schritt c) mit dem erzeugten Datenabfragemuster assoziiert.

Das Datenauswertemodul 30 ist ausgebildet, eine Gruppe der Datensätze von verstorben Körpern mit einer nicht hinreichenden Übereinstimmung mit einer Gruppe der Datensätze von verstorbenen Körpern mit einer hinreichenden Übereinstimmung, unter Berücksichtigung der Datenabfragemuster, zu vergleichen und bei erkannten inhärenten Datenabfragemustern die Datensätze der verstorbenen Körper mit einer nicht hinreichenden Übereinstimmung mittels eines mathematischen Modells, welches es erlaubt zu mindestens teilweise, die Werte oder Wertintervalle der unvollständigen Kategorien / Daten zu vervollständigen. Die ausgewählten und geprüften Daten D werden gemäss dem Schritt d) bereitgestellt.

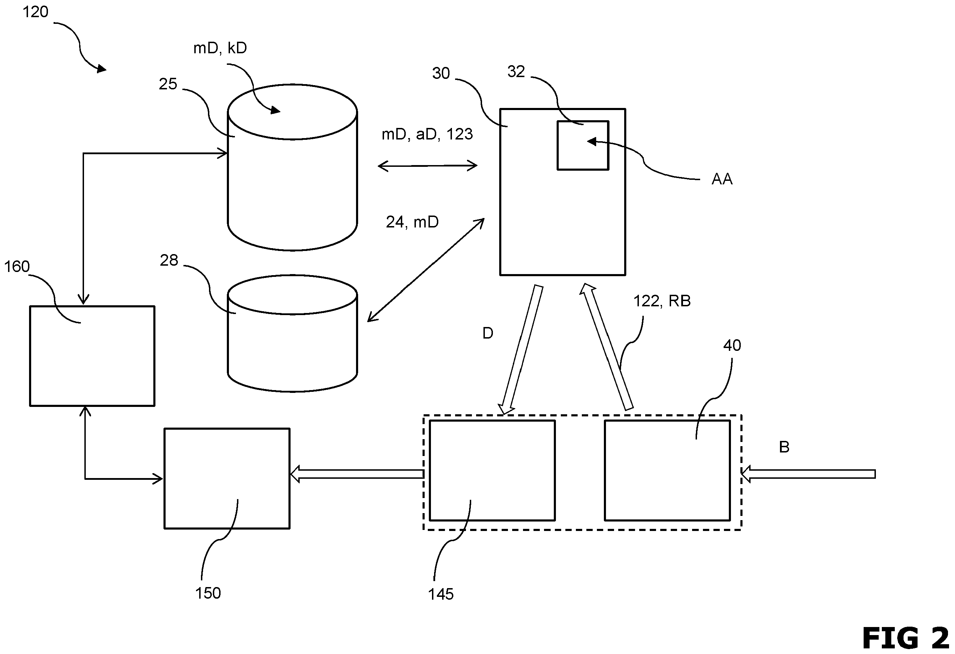

**Figur 2** zeigt eine weitere Ausführungsform eines Systems 120 zur Identifikation von medizinischen Daten mD auf Basis von einer Zieldefinition 122, wobei das System 120 im Grunde gleich funktional und strukturell aufgebaut ist, wie das System 20 gemäss der **Figur 1****.** Das System 120 umfasst zusätzlich ein KI-Modul 150 und eine medizinische Untersuchungseinrichtung zur medizinisches Datengewinnung 160. Das KI-Modul 150 ist mit der medizinischen Untersuchungseinrichtung 160 zum Austausch von Daten und/oder von Steuerbefehlen verbunden.

Die Zieldefinition 122 umfasst hier eine Fragestellung sowie medizinische Daten mD. Die analysierte Zieldefinition 123 wird dem Auswahlalgorithmus zur Verfügung gestellt, um fehlende medizinische Daten mD auszuwählen. Die Fragestellung ist mittels einer Analyse von medizinischen Daten mD von verstorbenen menschlichen Körpern aus dem Datenspeichermedium 25 beantwortbar.

Beispielsweise umfasst die Zieldefinition 122 auch mindestens ein medizinisches Datengewinnungsverfahren. Es wäre denkbar, dass bei einem CT-Scan eines Leichnams ein künstliches Hüftgelenk detektiert wird, wobei die Erhebung von erweiterten medizinischen Daten mD mit der Untersuchungseinrichtung zur medizinisches Datengewinnung 160 mithilfe einer Probenentnahme im Bereich des künstlichen Hüftgelenks erfolgt.

In dieser Ausführungsform werden die ausgewählten und überprüften Daten D an der Schnittstelle 45 einem Künstliche Intelligenz (KI)-Modul 150 übermittelt. Ein Benutzer B ist für die Ausgabe der ausgewählten oder überprüften Daten D nicht zwingend notwendig.

Das Bereitstellen der ausgewählten und überprüften Daten D ermöglicht es dem KI -Modul 150 zu entscheiden, ob die Daten D der eingegebenen Zieldefinition 122 entsprechen, wobei die folgenden Schlussfolgerungen bzw. Erkenntnisse folgen: Entweder wurden die zur Erfüllung der Zieldefinition 122 geeigneten medizinischen Daten mD mit der gewünschten Relevanz ausgegeben, oder es werden Informationen und/oder Instruktionen zum durchgeführten Vervollständigungsprozess bereitgestellt und/oder es werden Informationen zu zukünftigen Datenerhebungen mit der Untersuchungseinrichtung 160 bereitgestellt. Beispielsweise gibt die analysierte Zieldefinition 123 vor, dass das Bereitstellen der medizinischen Daten mD im Schritt d) nur erfolgen kann, wenn die Anzahl der medizinischen Daten mD mit der gewünschten medizinischen Relevanz einen vorgegebenen Schwellwert von 98%, erreicht ist.

Das KI-Modul 150 ist wiederum ausgebildet, den medizinischen Daten mD eine medizinische Relevanz auf Basis der Zieldefinition 122 zuzuweisen und verknüpft somit die medizinischen Daten mD mit unterschiedlichen Zieldefinitionen 122. Das KI-Modul 150 ist weiters ausgebildet, den medizinischen Daten mD eine medizinische Relevanz zuzuordnen, wenn nötig.

Das Datenauswertemodul 30 ist ausgebildet, eine Gruppe der Datensätze von verstorben Körpern mit einer nicht hinreichenden Übereinstimmung mit einer Gruppe der Datensätze von verstorbenen Körpern mit einer hinreichenden Übereinstimmung, unter Berücksichtigung der Datenabfragemuster, zu vergleichen und bei erkannten inhärenten Datenabfragemustern die Datensätze der verstorbenen Körper mit einer nicht hinreichenden Übereinstimmung mittels eines mathematischen Modells, welches es erlaubt zu mindestens teilweise, die Werte oder Wertintervalle der unvollständigen Kategorien / Daten zu vervollständigen. Dabei kann das Datenauswertemodul 30 über die Schnittstelle 145 auf das obenstehende KI-Modul 150 zugreifen oder eine eigene künstliche Intelligenz umfassen, welche beispielsweise in der Recheneinheit 32 angeordnet ist.

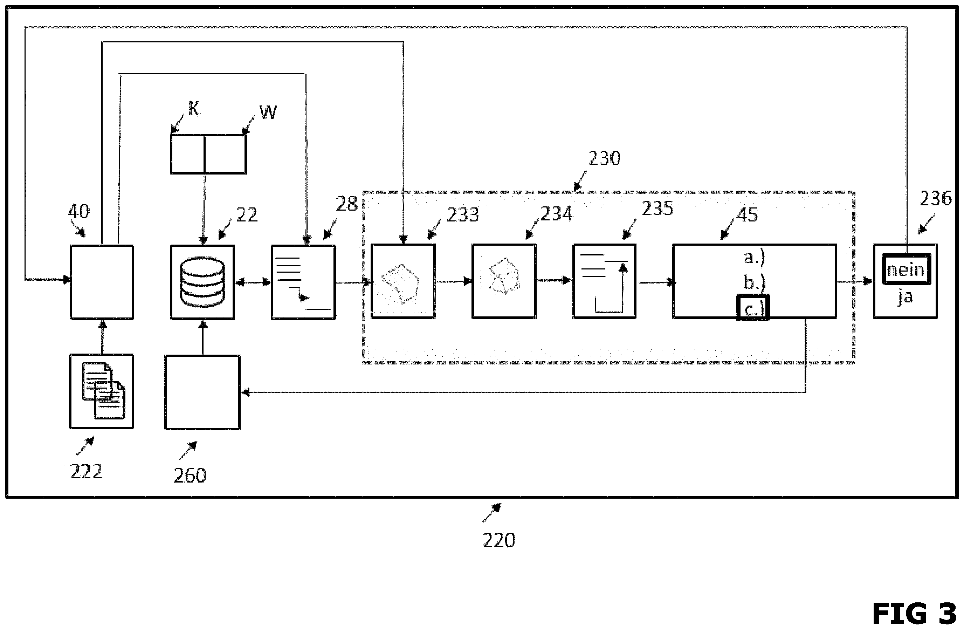

**Figur 3** zeigt eine weitere Ausführungsform eines Systems 220 zur Identifikation von medizinischen Daten mD auf Basis von einer Zieldefinition 222, wobei das System 220 weitgehend gleich funktional und strukturell aufgebaut ist, wie eines der Systeme 20, oder 120 gemäss **Figur 1** oder **Figur 2****.** Eine Ausführungsform des Datenauswertemoduls 230 wird hier etwas detaillierter offenbart und umfasst eine selbstlernende Klassifikations- und Mustererzeugungseinheit 233, eine Mustervergleichseinheit 234, eine Autovervollständigungseinheit 235. Weitere Ausführungsformen des Datenauswertemoduls enthalten nur einige der zuvor genannten Einheiten (nicht gezeigt).

Die Zielsetzung 222 umfasst eine Fragestellung und Rahmenbedingungen, mit denen die medizinischen Daten mD von verstorbenen Körpern aus einem Datenspeichermedium 25 identifiziert werden, welche geeignet sind, die Zieldefinition zu beantworten bzw. zu erfüllen. Im Datenspeichermedium 25 werden die Ergebnisse vergangener Autopsien mindestens eines Autopsiegerätes 260 und historische medizinische Daten von verstorbenen Körpern hinterlegt, wobei die medizinischen Daten der verstorbenen Körper durch eindeutige Kategoriebezeichnungen bzw. Kategorie K (z.B. Leukozyten) bzw. Kategorieebenenbezeichungen und dazugehörigen Werten bzw. Werteintervallen W beschrieben werden.

Ein Beispiel für die Fragestellung in der Zieldefinition 222 lautet: Gibt es Hinweise, dass die kontinuierliche Einnahme eines pharmazeutischen Präparates, in einer 40- bis 60-jährigen Patientengruppe zu Allergien führt.

In der selbstlernenden Expertendatenbank 28 sind die Zusammenhänge bzw. Verknüpfungen zwischen der in den Zieldefinitionen 222, bzw. den Segmenten der Zieldefinitionen formulierten Aussageforderung und der dazu auszuwertenden Kategorien der medizinischen Daten mD bzw. Datensätze der verstorbenen Körper hinterlegt, wobei jede Kategorie aus einer Kategoriebezeichnung (z.B. Leukozyten) und einem Wert bzw. Werteintervall besteht.

Die Fragestellung der Zieldefinition 222, welche über die Eingabeeinrichtung 40 elektronisch eingelesen wird, wird durch die selbstlernende Klassifikations- und Mustererzeugungseinheit 233 analysiert, sodass es möglich ist, unter Verwendung der Inhalte der selbstlernenden Expertendatenbank 28, einerseits die medizinischen Daten mD bzw. die Datensätze zu identifizieren, welche zur Beantwortung der Fragestellung geeignet sind, und andererseits die Segmente der Datensätze auszuwählen, welche die Informationen im Sinne der Fragestellung enthalten. Dabei bedient sich die Klassifikations- und Mustererzeugungseinheit 233 der Technologien und Methoden der Mustererzeugung und/oder Fuzzy-Logik und/oder neuronaler Netzwerke und übersendet die Ergebnisse an eine Mustervergleichseinheit 234.

Beispielsweise wird im Datenauswertemodul 30 erkannt, welche Parameter der Datensätze ausgewertet werden, um den Datensatz als relevant im Sinn der Fragestellung zu identifizieren und welche Parameter innerhalb eines Datensatzes analysiert werden, um eine Antwort auf die formulierte Fragestellung der Zieldefinition 222 zu geben. Beispielsweise erfolgt eine Identifikation von Datensätzen, welche auf eine kontinuierliche Einnahme eines pharmazeutischen Präparates schließen lassen und Auswertung von Parametern, welche Hinweise auf Allergien geben können, z.B. Immunglobuline und Auswertung des Immunglobulin E Wertes (IgE).

Die Mustervergleichseinheit 234 vergleicht das Datenabfragemuster mit den in dem Datenspeichermedium 25 hinterlegten medizinischen Daten mD bzw. Datensätzen und kommt nach Auswertung der jeweiligen Datensatrsegmente zu drei möglichen Ergebnissen, welche über eine Ausgabeeinheit 46 mittels eines Displays ausgegeben werden:
Ergebnis 1: Es werden medizinische Daten mD von verstorbenen Körpern mit einer hinreichenden, z.B. 100%-igen, Übereinstimmung identifiziert und ausgegeben, oder
Ergebnis 2: Es werden medizinische Daten mD von verstorbenen Körpern mit einer nicht hinreichenden Übereinstimmung, bezüglich der Kategorisierung, identifiziert. In diesem Zusammenhang wurden auch medizinische Daten mD identifiziert, welche geeignet sind, die Fragestellung zu beantworten, jedoch sind die jeweiligen Kategorienwerte nicht in den medizinischen Daten mD erfasst, oder
Ergebnis 3: Es werden medizinische Daten mD von verstorbenen Körpern mit einer unzureichenden Datenbasis identifiziert, so dass eine zukünftig erweiterte Datenerhebung gemäß eines Datenabfragemusters ausgelöst werden kann bzw. muss.

In Falle eines Ergebnisses 2, ist es möglich über eine Autovervollständigungseinheit 235 unvollständige Datensätze zu vervollständigen. Dazu wird in der Gruppe der Datensätze der medizinischen Daten mD mit hinreichender Übereinstimmung, unter Berücksichtigung der Datenabfragemuster, nach Datenmustern gesucht, welche in der Gruppe der Datensätze mit einer nicht hinreichenden Übereinstimmung wieder zu erkennen sind, um dann mittels eines mathematischen Modells, welches es erlaubt zu mindestens teilweise die Werte oder Wertintervalle der unvollständigen Kategorien / Variablen zu vervollständigen.

Darüber hinaus ist eine Rückkopplungseinheit, beispielsweise als KI-Modul 150, vorhanden, welche es ermöglicht, im Falle des Ergebnisses 3 die zukünftig nötigen Schritte des Autopsiegerätes 260 festzulegen.

Ebenso verfügt System 220 über eine Kundenzufriedenheits-Informationseinheit 236 welche einerseits hinsichtlich der Auswirkung der selbstlernenden Klassifikations- und Mustererzeugungseinheit 233, im Sinne der Verbesserung der Datenabfragemustergenerierung, und andererseits im Sinne der Verbesserung der Zusammenhänge der selbstlernenden Expertendatenbank 28 einen Beitrag leistet.

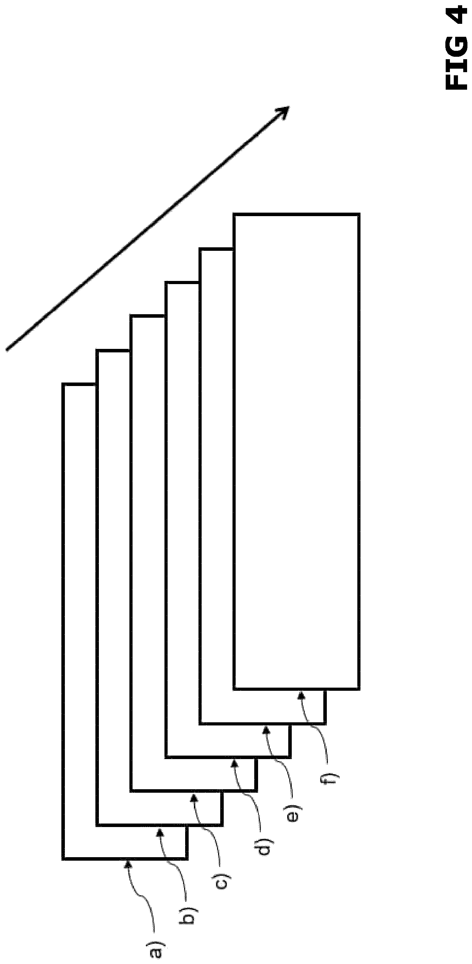

**Figur 4** zeigt eine schematische Darstellung des computerimplementierten Verfahrens zur Identifikation von medizinischen Daten auf Basis von Zieldefinitionen, umfassend mindestens die folgenden Schritte:
a) Bereitstellen von einzelnen oder mehreren medizinischen Daten aus einem Datenspeichermedium,
b) Erhalten von mindestens einer Zieldefinition in einem Datenauswertemodul mit einer Recheneinheit,
c) Analysieren der mindestens einen Zieldefinition in der Recheneinheit,
d) Auswählen von medizinischen Daten aus dem Datenspeichermedium mit einem Auswahlalgorithmus, wobei die ausgewählten Daten verknüpft mit der mindestens einen Zieldefinition ausgewählt werden,
e) Überprüfen einer vorgegebenen Vollständigkeit der medizinischen Daten mit einer gewünschten medizinischen Relevanz im Datenauswertemodul basierend auf der analysierten Zieldefinition,
f) Bereitstellen der ausgewählten und/oder überprüften Daten an einer Schnittstelle zur Bereitstellung für einen Benutzer und/oder ein KI-Modul, wobei insbesondere das Datenauswertemodul eine Benutzerschnittstelle veranlasst, die ausgewählten und/oder überprüften Daten an der Benutzerschnittstelle auszugeben.

Das zuvor genannte computerimplementiere Verfahren ist in einem hier offenbarten System 20, 120, 220 und insbesondere mit dem Datenauswertemodul 30 und mit zumindest einem Prozessor der Recheneinheit 32 ausführbar.

Die medizinischen Daten mD stammen unter anderem von einem verstorbenen menschlichen Körper und werden mithilfe eines Autopsie-Prozesses geschaffen.

Das Datenspeichermedium 30 umfasst ein hierarchisches Kategorienschema, mittels welchem die medizinischen Daten mD aus menschlichen Körpern strukturiert abgelegt werden können, wobei jeder Kategorie, bzw. Kategorie-Ebene eine eindeutige Kategoriebezeichnung (z.B. Leukozyten), bzw. Kategorieebenenbezeichung (z.B. Granulozyten) und einen Wert bzw. ein Werteintervall umfasst.

Darüber hinaus gibt es eine Liste von Fragestellungen mit zugehörigen zu erhebenden medizinischen und/oder körperbezogenen Daten kD, wobei einerseits die Liste der Fragestellungen im Verfahren laufend erweitert wird und andererseits die je Fragestellung zugehörigen zu erhebenden Daten im Verfahren laufend aktualisiert werden.

Im Schritt c) wird ein Datenabfragemuster mithilfe der analysierten Zieldefinition 23 erstellt, wobei Datenabfragemuster auf Grundlage bestimmter Fragestellungen generiert werden, und Daten aus dem Datenpool des Datenspeichermediums 25 ausgewählt und mit dem jeweiligen Datenabfragemuster verglichen werden.

Im Falle, dass die Übereinstimmung zwischen dem Datenabfragemuster und den medizinischen Daten mD aus menschlichen Körpern nicht vollständig bzw. nicht vorhanden ist, werden nach dem Schritt e) die aktuellen Daten aus menschlichen Körpern mit historischen Daten aus menschlichen Körpern angereichert. Die vorgegebene Vollständigkeit der medizinischen Daten lässt sich insbesondere verbessern, indem die unvollständigen Daten zuerst mit Daten der Patientenakte angereichert werden und, wenn notwendig, in einem nächsten Schritt auch medizinische Daten aus anderen menschlichen Körpern, die aufgrund statistischer Kriterien eine ausreichend hohe Ähnlichkeit mit dem betroffenen Fall besitzen, weiter angereichert werden. Auch dieser Prozess ist laufend optimierbar und mithilfe eines KI-Moduls weiter verbesserbar.

Des Weiteren ist die Recheneinheit 32 ausgebildet, zumindest die ausgewählten medizinischen Daten mD in Kategorien einzuteilen. Dabei umfasst das Datenabfragemuster einen Identifikationsteil, welcher die relevanten Daten anhand der Kategorien identifiziert, und einen Auswerteteil, welcher die medizinischen Daten der identifizierten Kategorien auswertet. Dabei werden die Werte der Daten in der identifizierten Kategorien mit der Recheneinheit ausgewertet. Dabei geht die Recheneinheit 32 mehrstufig vor, wobei in einem ersten Schritt die durch die mindestens eine Zieldefinition und/oder medizinischen Daten definierten Kategorien identifiziert werden und in einem zweiten Schritt die zur Beantwortung der mindestens einen Zieldefinition relevanten Werteintervalle festgelegt werden.

Der Auswahlalgorithmus AA ist ausgebildet, das erzeugte Datenabfragemuster mit den im Datenspeichermedium 35 hinterlegten medizinischen Daten mD zu vergleichen, um die vorgegebene Vollständigkeit der Daten im Schritt e) zu überprüfen. Dabei kann der Auswahlalgorithmus AA die Datenabfragemuster mit den im Datenspeichermedium 25 hinterlegten, kategorisierten Daten bzw. Datensätzen aus menschlichen Körpern vergleichen und (a) die medizinischen Daten bzw. Datensätze der menschlichen Körper mit einer hinreichenden, z.B. 100%-igen, Übereinstimmung ausgeben, oder (b) die medizinischen Daten bzw. Datensätze der menschlichen Körper mit einer nicht hinreichenden Übereinstimmung, bezüglich der Kategorisierung ausgeben (z.B. Werte auf bestimmten Kategorie-Ebenen liegen nicht vor), oder (c) bei einer unzureichenden Vollständigkeit eine zukünftige Identifikation bzw. Datenerhebung gemäss einem Datenabfragemuster auslösen, wobei sich die zukünftige Datenerhebung beispielsweise entweder auf einen nächsten Autopsieschritt einer laufenden Autopsie und/oder auf die Datenerhebungen von zukünftigen Autopsien bezieht. Dabei kann die Definition der zukünftigen Datenerhebung entweder mehrere alternative medizinische Daten enthalten, oder in Abhängigkeit der Auswahl mit dem Auswahlalgorithmus aus der Recheneinheit eine Reduzierung der erhobenen medizinischen Daten nach sich ziehen.

Ein Computerprogrammprodukt umfasst Programmbefehle, welche ausgebildet sind, zumindest ein hier vorliegend beschriebenes Verfahren auszuführen. Dabei ist das Computerprogrammprodukt in einer Recheneinheit 32 ausführbar, welche mit einem Datenauswertemodul verbunden ist oder darin integriert ist.

Die Programmbefehle veranlassen in einer möglichen Ausführungsform einen Prozessor und dessen Peripherie bei deren sequenziellen Abarbeitung zumindest die folgenden Schritte in den zuvor genannten Identifikationssystemen 20, 120, 220 abzubilden:
- Erstellung von Datenabfragemustern basierend auf mindestens einer Zieldefinition;
- Abgleich der Datenabfragemuster mit vorhandenen Daten aus menschlichen Körpern.
- Entscheidung, ob die analysierte Zieldefinitionen durch die vorhandenen Daten aus menschlichen Körpern erfüllt werden können oder ob eine weitere Erhebung von Autopsiedaten erforderlich ist.
- Entscheidung, welche und wie viele medizinische Daten aus menschlichen Körpern im Zweifelsfall zusätzlich identifiziert und erfasst werden müssen.

Darüber hinaus ist das Identifikationssystem selbstlemend, welches auf Basis der gewonnen Kenntnisse und der durchgeführten Datenbeschaffung das Identifikationssystem kontinuierlich weiterentwickelt und verbessert.

Ein computerlesbares Speichermedium umfasst das zumindest eine Computerprogrammprodukt, das bei der Ausführung durch zumindest eine Recheneinheit dieses veranlasst, zumindest eines der vorliegend beschriebenen Verfahren durchzuführen.

### Bezugszeichenliste

- 20: Identifikationssystem
- 22: Zieldefinition
- 23: analysierte Zieldefinition
- 24: historische Zieldefinition
- 25: Datenspeichermedium
- 28: Expertendatenbank
- 30: Datenauswertemodul
- 32: Recheneinheit
- 40: Eingabeeinrichtung
- 45: Benutzerschnittstelle
- 46: Ausgabeeinheit

- 120: Identifikationssystem
- 122: Zieldefinition
- 123: analysierte Zieldefinition
- 145: Schnittstelle
- 150: KI Modul
- 160: Untersuchungseinrichtung

- 220: Identifikationssystem
- 222: Zieldefinition
- 230: Datenauswertemodul
- 233: Klassifikations- und Mustererzeugungseinheit
- 234: Mustervergleichseinheit
- 235: Autovervollständigungseinheit
- 236: Kundenzufriedenheitsinformationseinheit
- 260: Autopsiegerät

- mD: medizinische Daten
- kD: körperbezogene Daten
- aD: ausgewählte Daten
- D: überprüfte Daten
- AA: Auswahlalgorithmus
- B: Benutzer
- ID: Identifikationsnummer
- K: Kategorien
- W: Werte/Werteintervalle
- RB: Rahmenbedingungen

## Patentansprüche

1. System (20; 120; 220) zur Identifikation von medizinischen Daten (mD) auf Basis von mindestens einer Zieldefinition (22; 122; 222) umfassend
ein Datenspeichermedium (25) mit mehreren medizinischen Daten (mD) zu menschlichen Körpern,
ein Datenauswertemodul (30; 230) mit einer Recheneinheit (32), welches zum Datenaustausch mit dem Datenspeichermedium (25) verbunden ist,
eine Eingabeeinrichtung (40) zum Eingeben mindestens einer Zieldefinition (22; 122; 222), welche mit dem Datenauswertemodul (30; 230) zum Datenaustausch verbunden ist, wobei eine Eingabe der mindestens einen Zieldefinition (22; 122; 222) das Datenauswertemodul (30; 230) dazu instruiert, mindestens die folgenden Schritte auszuführen:
a) Analysieren der mindestens einen Zieldefinition (22; 122; 222) in der Recheneinheit (32), insbesondere auf Basis mindestens einer bereits hinterlegten historischen Zieldefinition (24) aus einer Expertendatenbank (28),
b) Auswählen von medizinischen Daten (mD) aus dem Datenspeichermedium (25) mit einem Auswahlalgorithmus (AA), wobei die ausgewählten Daten verknüpft mit der mindestens einen analysierten Zieldefinition (23) ausgewählt werden, und insbesondere mit weiteren medizinischen Daten (mD) aus der Expertendatenbank (28) verglichen werden,
c) Überprüfen einer vorgegebenen Vollständigkeit der medizinischen Daten (mD) mit einer gewünschten medizinischen Relevanz im Datenauswertemodul (30; 230) basierend auf der analysierten Zieldefinition (23),
d) Bereitstellen der ausgewählten und/oder überprüften Daten (D) an einer Schnittstelle (45; 145) für einen Benutzer und/oder ein KI-Modul (150), wobei insbesondere das Datenauswertemodul (30; 230) die Schnittstelle (45; 145) veranlasst, die ausgewählten und/oder überprüften Daten an einer Benutzerschnittstelle (45; 145) auszugeben.

2. System nach Anspruch 1, **dadurch gekennzeichnet, dass** das Datenspeichermedium (25) körperbezogene Daten (kD) umfasst, welche mit den medizinischen Daten (mD) zumindest eines menschlichen Körpers assoziiert sind, und insbesondere die medizinischen Daten (mD) von einem verstorbenen menschlichen Körper stammen, wobei insbesondere die medizinischen Daten (mD) und/oder körperbezogenen Daten (kD) anonymisierte Daten sind.

3. System nach Anspruch 1, oder 2, **dadurch gekennzeichnet, dass** die mindestens eine Zieldefinition (22; 122; 222) mindestens eine Fragestellung und/oder medizinische Daten (mD) und/oder körperbezogene Daten (kD) und/oder mindestens ein medizinisches Datengewinnungsverfahren umfasst.

4. System nach einem der vorgenannten Ansprüche, **dadurch gekennzeichnet, dass** die ausgewählten medizinischen Daten (mD) und/oder körperbezogenen Daten (kD) in einer Datenstruktur im Datenspeichermedium (25) abgelegt sind.

5. System nach einem der vorgenannten Ansprüche, **dadurch gekennzeichnet, dass** die Recheneinheit (32) ausgebildet ist, im Schritt a) ein Datenabfragemuster mithilfe der analysierten Zieldefinition (23) zu erzeugen.

6. System nach Anspruch 5, **dadurch gekennzeichnet, dass** die Recheneinheit (32) ausgebildet ist, die medizinischen Daten (mD) und/oder die mindestens eine Zieldefinition (22; 122; 222) gemäss eines Kategorienschemas in einzelne Kategorien (K) des Kategorienschemas zerlegt und somit ein Datenabfragemuster erzeugt, und insbesondere ausgebildet ist, zumindest die ausgewählten medizinischen Daten (mD) in mindestens zwei Kategorien (K) einzuteilen.

7. System nach Anspruch 5 oder 6, **dadurch gekennzeichnet, dass** das Datenabfragemuster einen Identifikationsteil, welcher die relevanten Daten anhand der Kategorien (K) identifiziert, und insbesondere einen Auswerteteil, welcher die Daten und/oder Datenintervalle (W) der identifizierten Kategorien auswertet, umfasst.

8. System nach einem der Ansprüche 5 bis 7, **dadurch gekennzeichnet, dass** der Auswahlalgorithmus ausgebildet ist, das erzeugte Datenabfragemuster mit den im Datenspeichermedium (25) hinterlegten medizinischen Daten (mD) zu vergleichen, um die vorgegebene Vollständigkeit der Daten im Schritt c) zu überprüfen und/oder das Überprüfen einer vorgegebenen Vollständigkeit der medizinischen Daten (mD) mit einer gewünschten medizinischen Relevanz im Schritt c) mit dem erzeugten Datenabfragemuster assoziiert ist.

9. System nach einem der vorgenannten Ansprüche, **dadurch gekennzeichnet, dass** das Datenauswertemodul (30; 230) ausgebildet ist, auf Basis der mindestens einen Zieldefinitionen (22; 122; 222) in Kombination mit Information aus der Expertendatenbank (28) die zur Beantwortung der analysierten Zieldefinition (23) notwendigen Datensatzfelder auszuwerten.

10. System nach einem der vorgenannten Ansprüche, **dadurch gekennzeichnet, dass** das Datenauswertemodul (30; 230) ausgebildet ist, eine Datenstruktur mit medizinischen Daten (mD) und/oder körperbezogenen Daten (kD) zu ergänzen und insbesondere diese vervollständigten medizinische Daten in einer weiteren Datenstruktur im Datenspeichermedium (25) abzuspeichern, insbesondere anonymisiert abzuspeichern.

11. System nach einem der vorgenannten Ansprüche, **dadurch gekennzeichnet, dass** mindesten eine medizinische Untersuchungseinrichtung (160; 260) vorhanden ist, welche mit der Schnittstelle (45; 145)und/oder mit dem KI-Modul zum Austausch von Daten (D) und/oder von Steuerbefehlen verbunden ist.

12. System nach einem der vorgenannten Ansprüche, **dadurch gekennzeichnet, dass** das Datenauswertemodul (30; 230) ausgebildet ist, Steuerbefehle für zumindest eine medizinische Untersuchungseinrichtung (160; 260) zu erstellen.

13. Computerimplementiertes Verfahren zur Identifikation von medizinischen Daten (mD)auf Basis von Zieldefinitionen (22; 122; 222; 23; 24) umfassend mindestens die folgenden Schritte:
a) Bereitstellen von einzelnen oder mehreren medizinischen Daten (mD) aus einem Datenspeichermedium (25),
b) Erhalten von mindestens einer Zieldefinition (22; 122; 222) in einem Datenauswertemodul (30; 230) mit einer Recheneinheit (32),
c) Analysieren der mindestens einen Zieldefinition (22; 122; 222) in der Recheneinheit (32), insbesondere auf Basis mindestens einer bereits hinterlegten historischen Zieldefinition (24) aus einer Expertendatenbank (28)
d) Auswählen von medizinischen Daten (mD) aus dem Datenspeichermedium (25) mit einem Auswahlalgorithmus (AA), wobei die ausgewählten Daten verknüpft mit der mindestens einen Zieldefinition (22; 122; 222) ausgewählt werden, und insbesondere mit weiteren medizinischen Daten (mD) aus der Expertendatenbank (28) verglichen werden,
e) Überprüfen einer vorgegebenen Vollständigkeit der medizinischen Daten (mD)mit einer gewünschten medizinischen Relevanz im Datenauswertemodul (30; 230) basierend auf der analysierten Zieldefinition (23),
f) Bereitstellen der ausgewählten und/oder überprüften Daten (D) an einer Schnittstelle (45; 145) zur Bereitstellung für einen Benutzer und/oder einem KI-Modul, wobei insbesondere das Datenauswertemodul (30; 230) die Schnittstelle (45; 145) veranlasst, die ausgewählten und/oder überprüften Daten (D) an einer Benutzerschnittstelle (45; 145) auszugeben.

14. Computerprogrammprodukt umfassend Programmbefehle, welche ausgebildet sind zumindest ein Verfahren nach Anspruch 13 auszuführen.

15. Computerlesbares Speichermedium, das zumindest ein Computerprogrammprodukt umfasst, das bei der Ausführung durch zumindest eine Recheneinheit (32) dieses veranlasst, zumindest ein Verfahren nach Anspruch 13 durchzuführen.
